(19) Europäisches Patentamt
European Patent Office
Office européen des brevets

(11) Veröffentlichungsnummer: **0 073 011**
**B1**

(12) # EUROPÄISCHE PATENTSCHRIFT

(45) Veröffentlichungstag der Patentschrift: **02.04.86**

(51) Int. Cl.[4]: **C 07 C 121/80**, A 61 K 31/275, C 07 C 120/00

(21) Anmeldenummer: **82107519.9**

(22) Anmeldetag: **18.08.82**

(54) **Neue 1-Aryloxy-3-alkinylamino-2-propanole und Verfahren zu ihrer Herstellung.**

(30) Priorität: **26.08.81 DE 3133719**

(43) Veröffentlichungstag der Anmeldung: **02.03.83 Patentblatt 83/9**

(45) Bekanntmachung des Hinweises auf die Patenterteilung: **02.04.86 Patentblatt 86/14**

(84) Benannte Vertragsstaaten: **AT BE CH DE FR GB IT LI LU NL SE**

(56) Entgegenhaltungen:
**EP - A - 0 035 733**
**EP - A - 0 035 734**
**CH - A - 516 511**
**DE - A - 2 503 222**
**FR - A - 2 218 900**

**Die Akte enthält technische Angaben, die nach dem Eingang der Anmeldung eingereicht wurden und die nicht in dieser Patentschrift enthalten sind.**

(73) Patentinhaber: **BOEHRINGER INGELHEIM KG, D-6507 Ingelheim am Rhein (DE)**

(72) Erfinder: **Köppe, Herbert, Dr., Neuweg 72, D-6507 Ingelheim (DE)**
Erfinder: **Kummer, Werner, Dr., Georg-Scheuing-Strasse 15, D-6507 Ingelheim (DE)**
Erfinder: **Stähle, Helmut, Dr., Rotweinstrasse 23, D-6507 Ingelheim (DE)**
Erfinder: **Muacevic, Gojko, Dr., In der Dörrwiese 13, D-6507 Ingelheim (DE)**
Erfinder: **Traunecker, Werner, Dr., Birkenweg 1, D-6531 Münster-Sarmsheim (DE)**

**EP 0 073 011 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Verbindungen der Formel

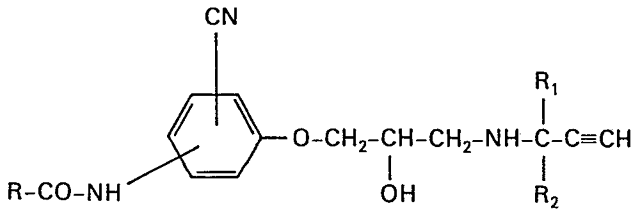

I

in der
R einen Cycloalkylrest mit 3 bis 6 C-Atomen, einen Phenylrest, der durch Methyl, Methoxi, Chlor oder Fluor substituiert sein kann, oder einen Phenoximethylrest, der durch Chlor oder Methyl substituiert sein kann,
$R_1$ und $R_2$ die gleich oder verschieden sein können, eine Methyl- oder Ethyl-, oder zusammen eine Alkylengruppe mit 4 bis 6 C-Atomen bedeutet, und deren Säureadditionssalze.

Die neuen Verbindungen können auf folgende Weise hergestellt werden:

a) Umsetzung einer Verbindung der allgemeinen Formel II

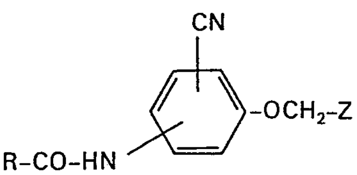

II

in der R wie in der Formel I definiert ist und Z die Gruppe

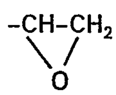

oder $-CHOH-CH_2-Hal$ (Hal = Halogen) bedeutet, mit einem Amin der allgemeinen Formel

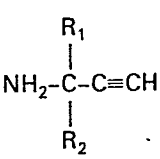

III

in der $R_1$ und $R_2$ die in Formel I angegebene Bedeutung haben.

b) Hydrolyse eines Oxazolidin-Derivats der allgemeinen Formel

IV

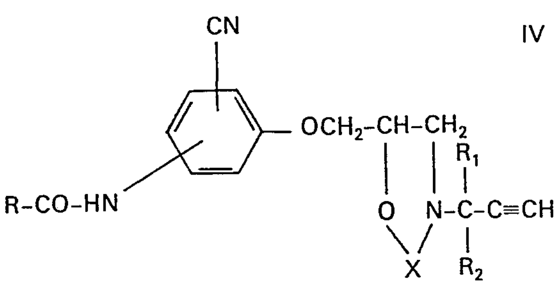

in der R bis $R_2$ wie in Formel I definiert sind und X eine $-CO-$, $-CH_2-$ oder -CH-niederalkyl-Gruppe bedeutet, beispielsweise mit Natron- oder Kalilauge in Wasser oder in einer Alkohol-Wasser-Mischung.

c) Umsetzung einer Verbindung der allgemeinen Formel V

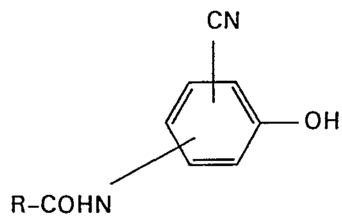

V

in welcher R die obengenannte Bedeutung hat bzw. eines Salzes dieses Phenols, mit einem Azetidinolderivat der allgemeinen Formel VI

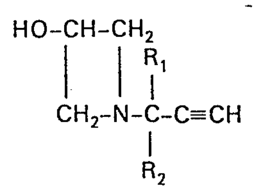

VI

in welcher $R_1$ und $R_2$ die obengenannte Bedeutung haben, in wasserfreiem Medium.

Die Oxazolidinone der Formel IV (d.h. Verbindungen, bei denen X = -CO- darstellt) sind beispielsweise ausgehend von den Epoxiden der Formel II herstellbar, indem man letztere mit einem (aus Chlorameisensäureäthylester und einem Amin der Formel III darstellbaren) Urethan der Formel VII

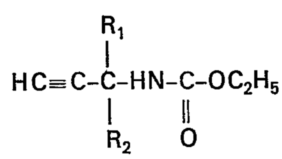

VII

in der $R_1$ und $R_2$ die obenbezeichnete Bedeutung haben, umsetzt.

Die Ausgangsphenole der allgemeinen Formel V und die Azetidinole der allgemeinen Formel VI können nach literaturbekannten Methoden (letztere s. beispielsweise Chem. pharm. Bull. (Japan), Vo. 22 (7), 1974, Seite 1490) hergestellt werden.

Die erfindungsgemässen Verbindungen besitzen ein asymmetrisches C-Atom an der CHOH-Gruppe und kommen daher als Racemat wie auch in Form der optischen Antipoden vor. Letztere können ausser durch Racematentrennung mit üblichen Hilfssäuren wie Dibenzoyl- (bzw. Dip-Toluyl-) D-Weinsäure oder D-3-Bromcampher-8-sulfonsäure auch durch Einsetzen von optisch aktivem Ausgangsmaterial erhalten werden.

Die erfindungsgemässen 1-Phenoxy-3-alkinyl-amino-2-propanole der allgemeinen Formel I können in üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure, Weinsäure oder 8-Chlortheophyllin.

Die Verbindungen der allgemeinen Formel I bzw. deren physiologisch verträgliche Säureadditionssalze haben im Tierversuch wertvolle therapeutische, insbesondere β-adrenolytische Eigenschaften gezeigt und können daher beispielsweise zur Behandlung oder Prophylaxe koronarer Herzkrankheiten, insbesondere der Angina pectoris und zur Behandlung von Herzarrhythmien, insbesondere von Tachycardien, in der Humanmedizin eingesetzt werden.

Therapeutisch besonders interessant sind die blutdrucksenkenden Eigenschaften der Verbindungen der Formel I. Zudem entfalten sie günstige metabolische und calciumantagonistische Eigenschaften. Die Verbindungen haben gegenüber bekannten β-Rezeptorenblockern, z.B. dem ähnlich strukturierten Handelsprodukt 1-(2-Acetyl-4-butyroylaminophenoxy)-3-isopropylamino-2-propanol den Vorteil beträchtlich verminderter Toxizität, besserer Wirkung und hervorragender Organselektivität.

Die Messung dieser Parameter erfolgte dabei nach den folgenden Vorschriften:

1. Hemmung der Isoprenalintachycardie (aludrinantagonistische Wirkung)

Methode: Hemmung der tachycarden Reaktion auf eine Standard-Dosis Isoprenalin und Einfluss auf die basale Herzfrequenz durch steigende i.v. Dosen eines β-Adrenolytikums.

Tiermaterial: Meerschweinchen beiderlei Geschlechts mit Körpergewichten von 270–350 g, Gruppenhaltung, Standard-Kost und Wasser bis zum Versuchsbeginn ad libitum. 16 Stunden vor dem Versuchsanfang Futterentzug.

Narkose: Äthylurethan 1,75 g/kg als 20%ige Lösung intraperitoneal, gegebenenfalls wurde nachinjiziert.

Präparation: Kanülierung einer Vena jugularis exterior für intravenöse Injektionen: Einbinden einer Trachealkanüle und künstliche Beatmung; subcutane Nadelelektroden zur Aufnahme des EKG, in der Regel Extremitätenableitung II, Registriergeschwindigkeit 25 mm/sec; Rektalthermometer zur Kontrolle der Körpertemperatur, die mit einer Wärmelampe (Infrarotstrahler) auf 34 bis 36 °C mit Hilfe einer elektronischen Automatikeinrichtung konstant gehalten wird.

Versuchsablauf: Die Herzfrequenz wird durch Auszählen der R-Zacken im EKG bestimmt, jeweils aus einer 3 bis 4 sec langen Registrierzeit. Etwa 30 min nach der Präparation wird im Abstand von 2 min 5 mal die normale Herzfrequenz gemessen und gemittelt. Anschliessend wird als adrenerges Stimulans 1 µg/kg Isoprenalin i.v. injiziert und danach 3 min lang alle 30 sec die Herzfrequenz erneut registriert. Die Isoprenalininjektionen werden während des ganzen Versuches im Abstand von 30 min wiederholt. Bleibt die Spontanfrequenz etwa konstant und ist die tachycarde Reaktion auf die ersten 2 bis 3 Isoprenalin-Gaben gleichmässig, dann wird 15 min nach der letzten und 15 min vor der nächsten Isoprenalin-Reaktion die erste Dosis der Prüfsubstanz i.v. injiziert. Weitere in geometrischer Reihe ansteigende Dosen der Prüfsubstanz folgen in Abständen von 60 min bis eine markante Hemmung der Isoprenalin-Tachycardie erreicht ist.

2. Prüfung auf Cardioselektivität am wachen Meerschweinchen

Prinzip: Nach der Methode von D. Dunlop und R.G. Shanks (Brit. J. Pharmacol. 32, 201 (1968) werden wache Meerschweinchen einer tödlichen Dosis eines Histaminaerosols ausgesetzt. Durch Vorbehandlung mit Isoprenalin werden die Tiere vor der letalen Wirkung des Histamins geschützt. Ein β-Adrenolytikum hebt die Isoprenalinwirkung auf, so dass der Schutz vor dem Histaminbronchospasmus verloren geht, falls es sich um eine nicht cardio-selektive Substanz handelt. Zeigt eine am Herzen wirksame β-adrenolytische Substanz in diesem Test keinen Antagonismus gegen Isoprenalin, dann kann Cardioselektivität (für sog. $\beta_1$-Rezeptoren) angenommen werden.

Tiermaterial: Meerschweinchen beiderlei Geschlechts (6 Tiere pro Dosis), mit 350 bis 400 g Körpergewicht, Gruppenhaltung. Standard-Futter und Wasser bis zum Versuchsbeginn ad libitum. 16 Stunden vor dem Versuchsanfang Futterentzug.

Versuchsablauf: Gruppen von je 6 Tieren (3 männliche + 3 weibliche) werden s.c. mit 5 oder mehr verschiedenen Dosen des β-Adrenolytikums behandelt. Fünfzehn Minuten später erhalten sie 0,1 mg/kg Isoprenalin contralateral s.c. injiziert. Nach weiteren 15 Minuten werden die Tiere in die zylindrische Kammer von 2 Liter Inhalt gesetzt, 45 Sekunden lang einem wässrigen Histaminaerosol (1,25%ig) ausgesetzt und anschliessend wird die Mortalität ausgewertet.

Auswertung: Die Letalität wird gegen den Logarithmus der Dosis aufgetragen und die $LD_{50}$ nach J. Litchfield und F. Wilcoxon (J. Pharmacol. Exp. Therap. 96, 99–113, 1949) ermittelt. Mit der $LD_{50}$ aus diesem Versuch und der cardialen $ED_{50}$ aus dem Versuch der Hemmung der Isoprenalintachycardie (narkot. Meerschweinchen) wird ein Selektivitätsquotient $\left(\frac{LD\,50}{ED\,50}\right)$ gebildet. Eine Substanz wird als cardioselektiv angesehen, wenn der Quotient grösser als 1 ist.

Als wertvoll haben sich dabei insbesondere solche Verbindungen der allgemeinen Formel I herausgestellt, bei denen $R_1$ und $R_2$ jeweils eine Methylgruppe darstellen (substituierte p-Acylamino-1-Phenoxy-3-(2-methylbutinyl-3-amino-2)-2-propanole). Besonders wertvoll ist insbesondere das 1-[2-Cyano-4-(4′-fluorbenzoyl)-aminophenoxi]-3-(2-methylbutinyl-3-amino-2)-2-propanol bzw. seine Salze.

Die Einzeldosis der erfindungsgemässen Substanzen liegt bei 1 bis 300 mg, vorzugsweise 5 bis 100 mg (oral) bzw. 1 bis 20 mg (parenteral).

Die erfindungsgemässen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Drageehülle zur Erzielung eines Depoteffekts aus mehreren Schichten bestehen, wobei die oben bei den Tabletten erwähnten Hilfsstoffe verwendet werden können.

Säfte der erfindungsgemässen Wirkstoffe bzw. Wirkstoffkombinationen können zusätzlich noch ein Süssungsmittel, wie Saccharin, Cyclamat, Glycerin oder Zucker, sowie ein geschmackverbesserndes Mittel, z.B. Aromastoffe, wie Vanillin oder Orangenextrakt, enthalten.

Sie können ausserdem Suspendierhilfsstoffe oder Dickungsmittel, wie Natriumcarboxymethyl-cellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemässen Verbindungen sind auch für die Kombination mit anderen pharmakodynamisch wirksamen Stoffen wie z.B. Coronardilatatoren, Sympathicomimetica, Herzglykosiden oder Tranquilizern geeignet.

Die folgenden Beispiele erläutern die Erfindung, ohne sie zu beschränken:

A. Herstellungsbeispiele

Beispiel 1

1-[2-Cyano-4-(2-chlorphenoxyacetylamino)-phenoxy]-3-(2-methylbutinyl-3-amino-2)-2-propanol · HCl

15,7 g (0,047 Mol) 1-[2-Cyano-4-(2-chlorphenoxyacetylamino)-phenoxy]-2,3-epoxypropan den in 80 ml Äthanol gelöst und nach Zugabe von 6,3 ml (0,063 Mol) 2-Methylbutin-3-amin-2 drei Stunden unter Rückfluss zum Sieden erhitzt. Nach Abdestillieren des Lösungsmittels wird mit HCl angesäuert und mit Äther gewaschen. Die wässrige Säure-Phase wird mit NaOH alkalisch gestellt und die ausfallende Base mit Methylenchlorid aufgenommen. Nach Trocknen über $Na_2SO_4$ wird das Methylenchlorid abdestilliert, der verbleibende Rückstand aus Essigester unter Zugabe von n-Hexan umkristallisiert und der Vorgang nochmals mit den gleichen Lösungsmitteln wiederholt. Die Base wird in Acetonitril gelöst, methanolische HCl und etwas Äther zugegeben und die Kristallisation durch Abkühlung eingeleitet, Ausbeute: 4,3 g, Fp: 172–175 °C.

Beispiel 2

1-(2-Cyano-4-cyclobutancarbonylaminophenoxy)-3-(2-methylbutinyl-3-amino-2)-2-propanol

7,5 g (0,024 Mol) 1-(2-Cyano-4-cyclobutancarbonylaminophenoxy)-2-hydroxy-3-chlorpropan werden in 20 ml Äthanol gelöst, 8,3 g (0,1 Mol 2-Methylbutin-3-amin-2 zugegeben und 3,5 Stunden zum Sieden unter Rückfluss erhitzt. Anschliessend wird das Äthanol abdestilliert und der verbleibende Rückstand mit $H_2O$ digeriert. Nach Ansäuern mit HCl wird mit Äther gewaschen, die saure wässrige Phase mit $NH_4OH$ alkalisch gestellt und die dabei ausfallende Base zweimal mit Essigester ausgeschüttelt. Die organische Phase wird mit $H_2O$ gewaschen, über $MgSO_4$ getrocknet und der Essigester i.V. abdestilliert. Der verbleibende kristalline Rückstand (Base) wird zweimal aus Acetonitril umkristallisiert, wobei 3,7 g Reinsubstanz gewonnen werden. Fp: 139–141 °C.

Beispiel 3

1-[2-Cyano-4-(4-fluorobenzoylamino)-phenoxy]-3-(2-methylbutinyl-3-amino-2)-2-propanol

9 g 1-[2-Cyano-4-(4-fluorobenzoyl-amino)-phenoxy]-3-chlor-2-propanol werden in 80 ml Äthanol gelöst und 12,5 ml 2-Methylbutin-3-amin-2 zugegeben. Nach 4stündigem Sieden unter Rückfluss wird das Lösungsmittel i.V. abdestilliert, der Rückstand mit verdünnter HCl angesäuert und mit Äther gewaschen. Die wässrige Phase wird mit $NH_4OH$ alkalisch gestellt, die ausfallende Base zweimal mit Essigester extrahiert, die organische Phase mit $H_2O$ gewaschen und über $MgSO_4$ getrocknet. Nach Abdestillieren des Essigesters wird der kristalline Rückstand zweimal aus Acetonitril umkristallisiert. Es werden 2,8 g farblose Kristalle der Base isoliert. Fp: 174–176 °C.

Nach der Vorschrift des Herstellungsbeispiels 2 wurden aus dem entsprechenden 1-(p-Acylaminophenoxy-2-hydroxy-3-chlorpropan der Formel II sowie dem entsprechenden Alkinylamin der Formel III durch Reaktion in Äthanol am Rückfluss noch folgende Verbindungen der allgemeinen Formel I synthetisiert (die Cyanogruppe steht jeweils in 2-Stellung des Phenylkerns):

| R (in 4-Position) | $R_1$ | $R_2$ | Fp, °C |
|---|---|---|---|
| 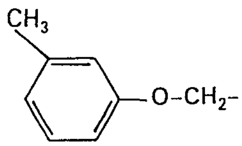 | $CH_3$ | $CH_3$ | 113–114° (Base) |
| 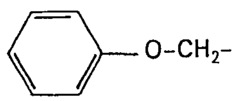 | $CH_3$ | $CH_3$ | 202–204° (Hydrochlorid) |
| 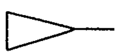 | $CH_3$ | $CH_3$ | 157–158° (Base) |
| 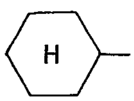 | $CH_3$ | $CH_3$ | 107–108° (Base) |
| 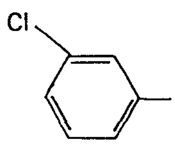 | $CH_3$ | $CH_3$ | 231–233° (Hydrochlorid) |
| 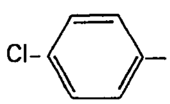 | $CH_3$ | $CH_3$ | 152–154° (Base) |
| 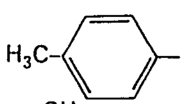 | $CH_3$ | $CH_3$ | 120–122° (Base) |
| 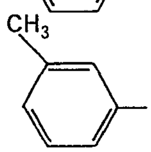 | $CH_3$ | $CH_3$ | 194–196° (Hydrochlorid) |
| 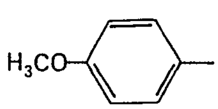 | $CH_3$ | $CH_3$ | 90– 92° (Base) |
| 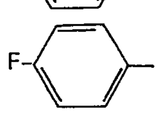 | $-(CH_2)_5$ | - | 120–123° (Base) |
| 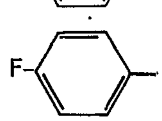 | $C_2H_5$ | $C_2H_5$ | 87– 89° (Base) |

B. Formulierungsbeispiele

1. Tabletten

| | |
|---|---|
| 1-[2-Cyano-4-(4'-fluorbenzoylamino)-phenoxy]-3-(2-methylbutinyl-3-amino-2)-2-propanol | 40,0 mg |
| Maisstärke | 164,0 mg |
| sek. Calciumphosphat | 240,0 mg |
| Magnesiumstearat | 1,0 mg |
| | 445,0 mg |

Herstellung:
Die einzelnen Bestandteile werden intensiv miteinander vermischt und die Mischung in üblicher Weise granuliert. Das Granulat wird zu Tabletten von 445 mg Gewicht verpresst, von denen jede 40 mg Wirkstoff enthält.

Anstelle des in diesem Beispiel genannten Wirkstoffs können auch die Substanzen 1-(2-Cyano-4-phenoxyacetylamino-phenoxy)-3-(2-methylbutinyl-3-amino-2)-2-propanol•hydrochlorid in gleicher Menge verwendet werden.

2. Gelatine-Kapseln
Der Inhalt der Kapsel setzt sich wie folgt zusammen:

| | |
|---|---|
| 1-[2-Cyano-4-(4'-fluorbenzoylamino)-phenoxy]-3-(2-methylbutinyl-3-amino-2-)-2-propanol•HCl | 25,0 mg |
| Maisstärke | 175,0 mg |
| | 200,0 mg |

Herstellung:
Die Bestandteile des Kapselinhalts werden intensiv vermischt und 200 mg-Portionen der Mischung werden in Gelatine-Kapseln geeigneter Grösse abgefüllt. Jede Kapsel enthält 25 mg des Wirkstoffs.

3. Injektionslösung
Die Lösung wird aus folgenden Bestandteilen hergestellt:

| | |
|---|---|
| 1-(2-Cyano-4-cyclobutancarbonylamino-phenoxy)-3-(2-methylbutinyl-3-amino-2)-2-propanol | 2,5 Teile |
| Natriumsalz der EDTA (Äthylendiamintetraessigsäure) | 0,2 Teile |
| dest. Wasser | ad 100,0 Teile |

Herstellung:
Der Wirkstoff und das EDTA-Salz werden in genügend Wasser gelöst und mit Wasser auf das gewünschte Volumen aufgefüllt. Die Lösung wird frei von suspendierten Partikeln filtriert und in 1 ccm-Ampullen unter aseptischen Bedingungen abgefüllt. Zuletzt werden die Ampullen sterilisiert und verschlossen. Jede Ampulle enthält 25 mg Wirkstoff.

4. Depotdragées

Kern:

| | |
|---|---|
| (-)-1-[2-Cyano-4-(4'-fluorbenzoylamino)-phenoxy]-3-(2-methylbutinyl-3-amino-2)-2-propanol•HCl | 25,0 g |
| Carboxymethylcellulose (CMC) | 295,0 g |
| Stearinsäure | 20,0 g |
| Celluloseacetatphtalat (CAP) | 40,0 g |
| | 380,0 g |

Herstellung:
Der Wirkstoff, die CMC und die Stearinsäure werden intensiv gemischt und die Mischung in üblicher Weise granuliert, wobei man eine Lösung des CAP in 200 ml eines Gemisches aus Äthanol: Äthylacetat verwendet. Das Granulat wird dann zu 380 mg-Kernen verpresst, die in üblicher Weise mit einer zuckerhaltigen 5%igen Lösung von Polyvinylpyrrolidon in Wasser überzogen werden. Jedes Dragée enthält 25 mg Wirkstoff.

5. Tabletten

| | |
|---|---|
| 1-[2-Cyano-4-(2'-chlorphenoxyacetylamino)-phenoxy]-3-(2-methylbutinyl-3-amino-2)-2-propanol-hydrochlorid | 35,0 g |
| 2,6-Bis-(diäthanolamino)-4,8-dipiperidinopyrimido-[5,4-d]-pyrimidin | 75,0 g |
| Milchzucker | 164,0 g |
| Maisstärke | 194,0 g |
| kolloidale Kieselsäure | 14,0 g |
| Polyvinylpyrrolidon | 6,0 g |
| Magnesiumstearat | 2,0 g |
| lösliche Stärke | 10,0 g |
| | 500,0 g |

Anstelle des in diesem Beispiel genannten β-adrenolytisch wirksamen Stoffs kann auch die Substanz 1-[2-Cyano-4-(4'-fluorbenzoyl-amino-phenoxy]-3-(3-äthylpentinyl-4-amino-3)-2-propanol in gleicher Menge verwendet werden.

Herstellung:
Der Wirkstoff wird zusammen mit dem Milchzucker, der Maisstärke, der kolloidalen Kieselsäure und dem Polyvinylpyrrolidon nach intensiver Durchmischung in üblicher Weise granuliert, wobei man eine wässrige Lösung der löslichen Stärke verwendet. Das Granulat wird mit dem Magnesiumstearat gemischt und zu 1000 Tabletten von je 500 mg Gewicht verpresst, die je 35 mg des ersten und 75 mg des zweiten Wirkstoffs enthalten.

**Patentansprüche**

1. Phenoxipropanolamine der allgemeinen Formel (I)

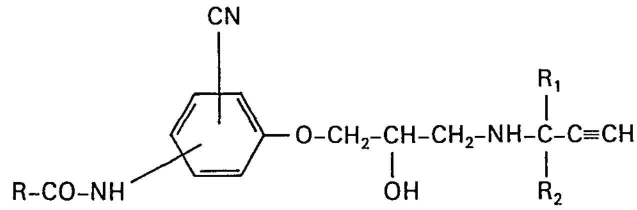

I

worin
R einen Cycloalkylrest mit 3 bis 6 C-Atomen oder einen Phenylrest, der durch Methyl, Methoxi, Chlor oder Fluor substituiert sein kann, oder einen Phenoximethylrest, der durch Chlor oder Methyl substituiert sein kann, und
$R_1$ und $R_2$, die gleich oder verschieden sein können, eine Methyl- oder Ethyl- oder zusammen eine Alkylengruppe mit 4 bis 6 C-Atomen bedeutet, und deren Säureadditionssalze.

2. 1-[2-Cyano-4-(4′-fluorbenzoyl-amino)-phenoxi]-3-(2-methylbutinyl-3-amino-2)-2-propanol bzw. seine Salze.

3. 1-[2-Cyano-4-(4′-fluorbenzoyl-amino-phenoxi]-3-(2-methylbutinyl-3-amino-2)-2-propanol-hydrochlorid.

4. Pharmazeutische Zubereitungen, enthaltend Substanzen der allgemeinen Formel (I) nach Anspruch 1 in Kombination mit üblichen Hilfs- und/oder Trägerstoffen.

5. Pharmazeutische Präparate, enthaltend Substanzen der allgemeinen Formel (I) nach Anspruch 1 in Kombination mit anderen pharmazeutischen Wirkstoffen sowie üblichen Hilfs- und/oder Trägerstoffen.

6. Verbindungen nach Anspruch 1 zur Verwendung als Wirkstoffe für Arzneimittel zur Behandlung und Prophylaxe von Erkrankungen der Herzkranzgefässe.

7. Verfahren zur Herstellung von pharmazeutischen Präparaten nach Anspruch 4 oder 5, dadurch gekennzeichnet, dass man Verbindungen der Formel (I) nach Anspruch 1 mit üblichen galenischen Hilfs- und/oder Trägerstoffen in üblichen pharmazeutischen Anwendungsformen verarbeitet.

8. Verfahren zur Herstellung von Verbindungen der allgemeinen Formel (I) nach Anspruch 1, dadurch gekennzeichnet, dass man

a) Verbindungen der allgemeinen Formel (II)

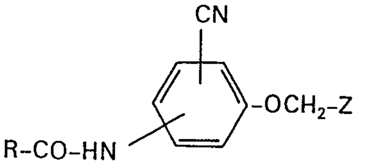

II

in der R wie in Formel I definiert ist und Z die Gruppe

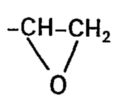

oder $-CHOH-CH_2-Hal$ (Hal = Halogen) bedeutet, mit einem Amin der allgemeinen Formel (III)

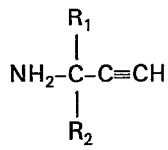

III

in der $R_1$ und $R_2$ wie in Formel (I) definiert sind, umsetzt oder dass man

b) ein Oxazolidin-Derivat der allgemeinen Formel (IV)

IV

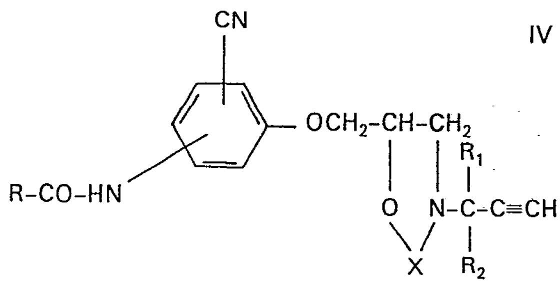

in der R, $R_1$ und $R_2$ wie in Formel (I) definiert sind und X eine $-CO-$, $-CH_2-$ oder -CH-Niederalkylgruppe bedeutet, hydrolysiert, oder dass man

c) eine Verbindung der allgemeinen Formel (V)

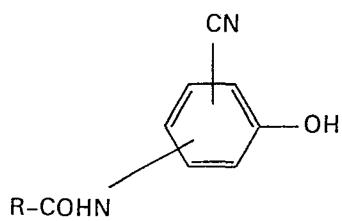

V

in welcher R wie in Formel (I) definiert ist (oder ein Salz dieser Verbindung) mit einen Azetidinolderivat der allgemeinen Formel (VI)

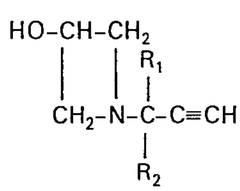

VI

in welcher $R_1$ und $R_2$ wie in Formel (I) definiert sind, umsetzt, und die nach einem der Verfahren

a) bis c) erhaltenen Verbindungen gewünschtenfalls in ihre Säureadditionssalze überführt.

## Claims

1. Phenoxypropanolamines of general formula

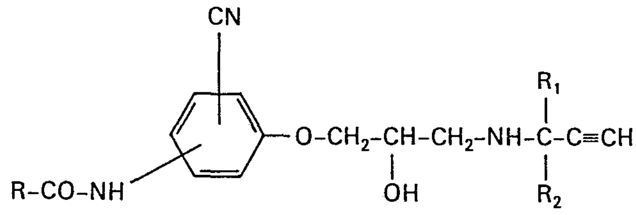

I

wherein
R represents a cycloalkyl group having 3 to 6 carbon atoms or a phenyl group which may be substituted by methyl, methoxy, chlorine or fluorine, or a phenoxymethyl group which may be substituted by chlorine or methyl, and
$R_1$ and $R_2$, which may be the same or different, represent a methyl or ethyl group or together represent an alkylene group with 4 to 6 carbon atoms, and the acid addition salts thereof.

2. 1-[2-Cyano-4-(4′-fluorobenzoyl-amino)-phenoxy]-3-(2-methylbutynyl-3-amino-2)-2-propanol or the salts thereof.

3. 1-[2-Cyano-4-(4′-fluorobenzoyl-amino-phenoxy]-3-(2-methylbutynyl-3-amino-2)-2-propanol hydrochloride.

4. Pharmaceutical preparations containing substances of general formula I as claimed in claim 1 together with conventional excipients and/or vehicles.

5. Pharmaceutical preparations containing substances of general formula I as claimed in claim 1 together with other pharmaceutical active substances and conventional excipients and/or vehicles.

6. Compounds as claimed in claim 1 for use as active substances for pharmaceutical compositions for the treatment and prophylaxis of diseases of the coronary blood vessels.

7. Process for preparing pharmaceutical preparations as claimed in claim 4 or 5, characterised in that compounds of formula I as claimed in claim 1 and processed with conventional galenic excipients and/or vehicles to produce conventional pharmaceutical preparations.

8. Process for preparing compounds of general formula I as claimed in claim 1, characterised in that

a) compounds of general formula II

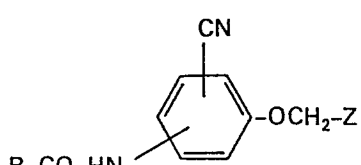

II

wherein R is defined as in formula I and Z represents the group

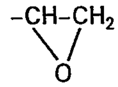

or $-CHOH-CH_2-Hal$ (Hal = halogen), are reacted with an amine of general formula III

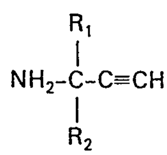

III

wherein $R_1$ and $R_2$ are defined as in formula I, or

b) an oxazolidine derivative of general formula IV

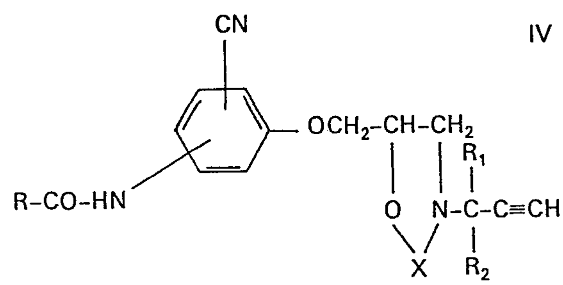

IV

wherein R, $R_1$ and $R_2$ are defined as in formula I and X represents a $-CO-$, $-CH_2-$ or -CH-lower alkyl group, is hydrolysed, or

c) a compound of general formula V

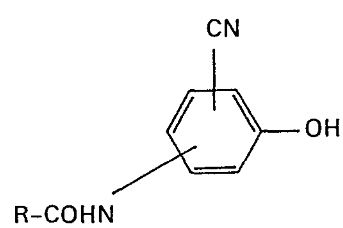

V

wherein R is defined as in formula I (or a salt of this compound) is reacted with an azetidinol derivative of general formula VI

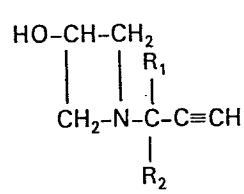

VI

wherein $R_1$ and $R_2$ are defined as in formula I, and if desired the compounds obtained according to one of the processes a) to c) are converted into the acid addition salts thereof.

## Revendications

1. Phénoxypropanolamines de formule générale (I)

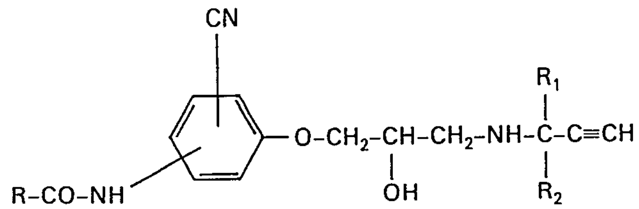

I

dans laquelle
R représente un radical cycloalcoyle avec 3 à 6 atomes de C ou un radical phényle qui peut être substitué par méthyle, méthoxy, chlore ou fluor, ou représente un radical phénoxyméthyle qui peut être substitué par chlore ou méthyle, et
$R_1$ et $R_2$ qui peuvent être identiques où différents, représentent un groupe méthyle ou éthyle ou ensemble un groupe alcoylène à 4 à 6 atomes de C, et leurs sels d'addtiton d'acides.

2. Le 1-[2-cyano-4-(4'-fluorobenzoyl-1-amino)-phénoxy]-3-(2-méthylbutynyl-3-amino-2)-2-propanol ou ses sels.

3. Le chlorhydrate de 1-[2-cyano-4-(4'-fluorobenzoyl-amino-phénoxy)-3-(2-méthyl-butynyl-3-amino-2)-2-propanol.

4. Préparations pharmaceutiques contenant des substances de formule générale (I) selon la revendication 1 en combinaison avec des adjuvants et/ou excipients usuels.

5. Préparations pharmaceutiques contenant des substances de formule générale (I) selon la revendication 1 en combinaison avec d'autres substances actives pharmaceutiques ainsi que des adjuvants et/ou excipients usuels.

6. Composés selon la revendication 1 pour l'utilisation en tant que substances actives pour des médicaments pour le traitement et la prophylaxie de maladies des vaisseaux coronaires.

7. Procédé pour la fabrication de préparations pharmaceutiques selon la revendication 4 ou 5, caractérisé en ce qu'on transforme des composés de formule (I) selon la revendication 1 au moyen d'adjuvants et/ou excipients galéniques usuels en des formes d'utilisation pharmaceutiques usuelles.

8. Procédé pour la préparation de composés de formule générale (I) selon la revendication 1, caractérisé en ce que

a) on fait réagir des composés de formule générale (II)

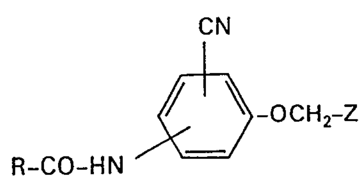

II

dans laquelle R est défini comme dans la formule I et Z représente le groupe

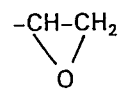

ou $-CHOH-CH_2-Hal$ (Hal = halogène), avec une amine de formule générale (III)

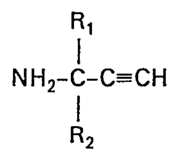

III

dans laquelle $R_1$ et $R_2$ sont définis comme dans la formule (I) ou en ce que

b) on hydrolyse un dérivé d'oxazolidine de formule générale (IV)

IV

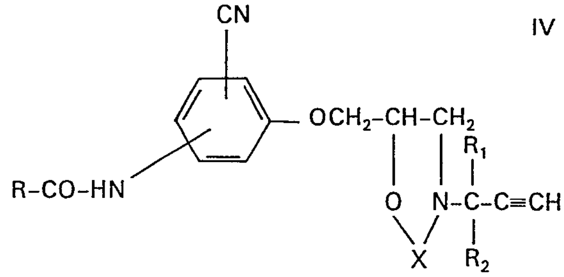

dans laquelle R, $R_1$ et $R_2$ sont définis comme dans la formule (I) et X représente un groupe -CO-, $-CH_2-$ ou -CH-alcoyle inférieur, ou en ce que

c) on fait réagir un composé de formule générale (V)

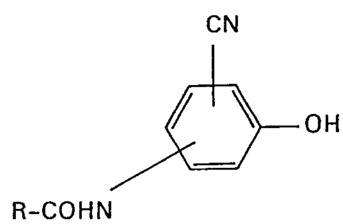

V

dans laquelle R est défini comme dans la formule (I) (ou un sel de ce composé) avec un dérivé d'azétidinol de formule générale (VI)

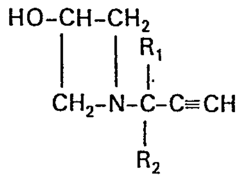

VI

dans laquelle $R_1$ et $R_2$ sont définis comme dans la formule (I),
et si on le désire on transforme les composés obtenus selon l'un des procédés a) à c) en leurs sels d'addition d'acides.